# EUROPÄISCHE PATENTSCHRIFT

(11) **EP 1 925 331 B1**
(45) Veröffentlichungstag und Bekanntmachung des Hinweises auf die Patenterteilung: **13.04.2016**
(21) Anmeldenummer: 08003371.5
(22) Anmeldetag: 16.02.2001
(51) Int. Cl.: A61M 16/08, A61M 16/10, F16L 59/02, F16L 53/00, A61M 16/16

(54) **Atemgasschlauchanordnung zur Zufuhr eines Atemgases**
Respiratory gas hose system for supplying a respiratory gas
Système flexible pour acheminer un gaz à respirer

(30) Priorität: 18.02.2000 DE 10007506
(43) Veröffentlichungstag der Anmeldung: 28.05.2008
(62) Teilanmeldung aus: 01909765.8
(73) Patentinhaber: ResMed R&D Germany GmbH, 82152 Martinsried (DE)
(72) Erfinder: Huber, Petra, 85435 Erding (DE); Lang, Bernd, 82166 Gräfelfing (DE); Biener, Achim, 85445 Aufkirchen (DE); Heidmann, Dieter, Castle Hill, NSW 2154 (AU)
(74) Vertreter: Vossius & Partner Patentanwälte Rechtsanwälte mbB

(56) Entgegenhaltungen:
- EP-A- 0 621 050
- WO-A-98/26826
- DE-U- 20 018 593
- GB-A- 2 338 420
- US-A- 2 516 864
- US-A- 3 856 051
- US-A- 4 121 583
- US-A- 4 400 420
- US-A- 4 722 334
- US-A- 5 377 670
- US-A- 5 600 752

## Beschreibung

Die Erfindung betrifft eine Atemgasschlauchanordnung zur Zufuhr eines Atemgases zu einer Person.

Derartige Atemgasschlauchanordnungen finden insbesondere im Bereich der Schlafmedizin zur Behandlung schlafbezogener Atmungsstörungen Anwendung. So kann beispielsweise bereits durch einen vergleichsweise geringen Atemgasüberdruck eine pneumatische Schienung der oberen Atemwege erreicht werden, wodurch auf wirkungsvolle Weise der Gefahr von Atemwegsobstruktionen vorgebeugt werden kann.

Die Behandlung schlafbezogener Atmungsstörungen durch eine kontinuierliche Überdruckbeatmung wird allgemein als CPAP-Therapie bezeichnet. Bei den hierzu verwendeten bekannten CPAP-Geräten ist üblicherweise in einem schallisolierten Gehäuse eine Fördereinrichtung, insbesondere ein Gebläse angeordnet, dessen Förderdruck, ggf. über eine elektronische Regeleinrichtung auf die Atmungstätigkeit des Patienten abgestimmt wird. Das durch die Fördereinrichtung geförderte Atemgas, in der Regel Umgebungsluft, wird über eine flexible Atemgasschlauchanordnung dem Patienten zugeführt. Hierzu werden im Regelfall Atemmasken verwendet, die auf die Nase des Patienten aufgesetzt werden, ohne hierbei den Mund des Patienten abzudecken. Es hat sich gezeigt, daß die CPAP-Therapie eine effektive und physiologisch gut verträgliche Therapiemethode zur Behandlung schlafbezogener Atmungsstörungen darstellt. Die zur Verbindung der patientenseitig angeordneten Atemmaske mit dem CPAP-Gerät vorgesehene flexible Schlauchleitung wird jedoch oft als störend empfunden.

US 4 121 583 A beschreibt eine tragbare Vorrichtung zur Zufuhr eines Atemgases umfassend ein Mundstück sowie einen Generator.

Der Erfindung liegt die Aufgabe zugrunde, eine Vorrichtung zur Zufuhr eines Atemgases zu einem Patienten zu schaffen, die sich durch einen erhöhten Anwendungskomfort auszeichnet.

Die Aufgabe wird durch ein Anschlusselement mit den Merkmalen des Anspruchs 1 gelöst. Vorteilhafte Ausgestaltungen des Anschlusselementes gemäß der Erfindung sind in den Unteransprüchen beschrieben. Eine erfindungsgemäße Atemgasschlauchanordnung zur Zufuhr eines Atemgases nach der Erfindung ist Gegenstand des unabhängigen Anspruchs 4. Vorteilhafte Ausgestaltungen der Atemgasschlauchanordnung sind in den Unteransprüchen angegeben. Vorrichtungen zur Zufuhr eines Atemgases geben die Ansprüchen 13 und 14 an.

Dadurch wird es auf vorteilhafte Weise möglich, einen unmittelbaren Berührungskontakt der Schlauchleitung mit dem Patienten zu vermeiden und die Schlauchleitung in einer unter ästhetischen Gesichtspunkten vorteilhaften Weise zu verbergen. In besonders vorteilhafter Weise wird auch der Kondensatbildung in der Schlauchleitung vorgebeugt, so daß auch im Falle hoher absoluter Feuchtigkeit des Atemgases sich im Inneren der flexiblen Schlauchleitung keine Kondensattropfen bilden können.

Gem. eines Beispiels ist der flexible Mantelkörper aus einem weichen Textilmaterial - insbesondere Fleecematerial - gefertigt.

Der flexible Mantelkörper ist in vorteilhafter Weise derart dimensioniert, daß die flexible Schlauchleitung darin zwanglos aufgenommen ist. Vorzugsweise beträgt der Innendurchmesser des flexiblen Mantelkörpers wenigstens das 1,3-Fache des Außendurchmessers der flexiblen Schlauchleitung. Durch das zwischen der Außenwandung der flexiblen Schlauchleitung und dem flexiblen Mantelkörper gebildete Luftpolster wird eine weitere Verbesserung des Anwendungskomforts sowie der Isolierwirkung erreicht.

Es ist möglich, den flexiblen Mantelkörper aus einem mehrlagigen Material zu bilden, das erst im Außenbereich eine unter ästhetischen Gesichtspunkten ausgewählte, angenehme Außenlage aufweist.

Der flexible Mantelkörper weist gem. eines Beispiels im Bereich seiner Enden eine Schließeinrichtung auf, durch welche der Endbereich des flexiblen Mantelkörpers verengt, beispielsweise eingeschnürt, und vergleichsweise festsitzend an der flexiblen Schlauchleitung fixiert werden kann. Als geeignete Fixiereinrichtung wird gem. einer besonders bevorzugten Ausführungsform der Erfindung eine Zurrschnur verwendet, die durch Ösen und/oder einen Hohlsaum hindurchgeführt ist und ein Zusammenschnüren des entsprechenden Endes des flexiblen Mantels ermöglicht. Alternativ hierzu oder auch in Kombination damit ist es möglich, im Endbereich des flexiblen Mantels eine Klettverschlußeinrichtung vorzusehen, über welche das Ende des flexiblen Mantelkörpers entsprechend verengt werden kann.

Die Wanddicke des flexiblen Mantelkörpers liegt vorzugsweise im Bereich von 1 - 7 mm. Das verwendete Material kann beispielsweise ein Vollmaterial (Vliesmaterial) oder auch ein mehrlagiges Material sein. In besonders vorteilhafter Weise ist eine flexible Textillage auf eine Polsterlage, beispielsweise aus Schaumstoffmaterial aufkaschiert.

Der flexible Mantelkörper ist vorzugsweise als schlauchartiger Körper ausgebildet und hierbei auf die flexible Schlauchleitung aufgeschoben.

Alternativ dazu ist es auch möglich, den flexiblen Mantelkörper aus einem Streifenmaterial zu bilden, das eine sich in dessen Längsrichtung erstreckende Verbindungseinrichtung aufweist. Diese Verbindungseinrichtung ist in vorteilhafter Weise durch ein Klettverschlußband gebildet. Alternativ hierzu sind auch Reißverschluß-Verbindungseinrichtungen oder auch Knopflochanordnungen sowie Druckknöpfe möglich.

In besonders vorteilhafter Weise ist es möglich, im Bereich des CPAP-Gerätes sowie vorzugsweise auch im Bereich der Atemmaske Verbindungsmittel vorzusehen, über welche der flexible Mantelkörper zuverlässig fixiert werden kann. In vorteilhafter Weise bestehen diese Fixiermittel aus einem Vlies- oder Kletthakenband, das im jeweiligen Endbereich (Maske oder CPAP-Gerät) am Schlauch fixiert vorzugsweise angeklebt oder geklemmt ist.

Es ist gemäß eines Beispiels auch möglich, im Bereich der Enden des Mantelkörpers elastische Bündchen vorzusehen durch welche das Mantelmaterial des Mantelkörpers auf den Schlauch oder die Schlauch-Endstücke gedrängt wird. Hierzu ist es beispielsweise möglich, ein Gummiband im Endbereich des Mantelkörpers vorzusehen. Das Gummiband ist hierbei vorzugsweise in einem Hohlsaum aufgenommen.

Es ist in vorteilhafter Weise möglich, im Bereich der Auflagezonen des Mantelkörpers auf dem Schlauch eine rutschfeste beispielsweise gummierte Lage vorzusehen.

Die Wärme- und Schallisolationswirkung kann in vorteilhafer Weise durch die Materialbeschaffenheit und den Aufbau des Mantelkörpers bedarfsgerecht abgestimmt werden. Eine besonders hohe Wärmeisolationswirkung wird erreicht, indem mehrere Lagen Fleece-Material verarbeitet werden. Es ist möglich, die Lagen des Fleece-Materiales vorzugsweise in Verbindung mit Deck- und Futterlagen abzusteppen oder zu quilten.

Zumindest der Außenbereich des Mantelkörpers ist vorzugsweise mehrfarbig ausgebildet. Diese Mehrfarbigkeit wird vorzugsweise durch Vernähen mehrfarbiger Textilabschnitte erreicht, wobei die Vebindungsnähte hierbei gleichzeitig als Quiltnähte dienen.

Es ist möglich, in den Mantelkörper eine Band- oder Stab-artige Versteifungseinlage einzubinden, so daß bei einer schlauchartigen Ausgestaltung des Mantelkörpers eine Aufzieh-Hilfe erreicht wird.

im Falle einer längsgeteilten Ausführungsform des Mantelkörpers ist es möglich, alternativ zu Klett- oder Knopf- insbesondere Druckknopf-Verbindungen hier einen Längsreißverschluß vorzusehen. Dieser Längsreißverschfuß ist vorzugsweise derart überlappt ausgebildet, daß die Reißverschlußstruktur nach außen überdeckt ist. Es ist auch möglich, hier eine Magnetverschlußeinrichtung vorzusehen.

Gemäß eines Beispiels ist in den Mantelkörper eine Heizeinrichtung integriert. Diese Heizeinrichtung kann durch eine elektrische Widerstandsheizung gebildet sein. Gemäß einer besonders bevorzugten Ausführungsform umfaßt die Heizeinrichtung eine Niedervoltheizfolie, wobei das Leistungsaufnahmeverhalten dieser Heizfolie derart gewählt ist, daß die Heizfolie eine vorgegebenen Heiztemperatur selbstregelnd nicht überschreitet. Die Heizfolie kann in den Mantelkörper eingenäht sein, wobei vorzugsweise das Wärmeisolationsvermögen des Mantelkörpers außerhalb der Heizfolie größer ist als im Bereich zwischen Heizfolie und Schlauch. Alternativ hierzu ist es auch möglich, den Schlauch als beheizbaren Atemgasschlauch auszubilden.

Die Spannungsversorgung der Heizeinrichtung erfolgt vorzugsweise über eine Niedervoltleitung die mit einem Anschlußstecker versehen ist, welcher in eine entsprechende Spannungsversorgungsbuchse eines Netzgerätes - oder vorzugsweise eine, an einem CPAP-Gerät, oder einem Luftbefeuchter vorgesehene Spannungsversorgungsdose einsteckbar ist. Durch einen derart beheizbar ausgebildeten Mantelkörper wird neben einer zuverlässigen Vermeidung von Kondensationserscheinungen auch Temperierung des Atemgases ermöglicht.

Es ist möglich, im Bereich des Mantelkörpers Isolationsmittel zur Wärmestrahlungsabschirmung vorzusehen. Vorzugsweise ist hierbei auf einer an die Schlauchleitung angrenzenden Innenseite des Mantelkörpers eine metallisierte beispielsweise Aluminium, Silber- oder Gold-bedampfte Superisolationsfolie vorgesehen.

Gemäß eines Beispiels sind an dem Mantelkörper wenigstens eine - vorzugsweise mehrere Fixierstellen vorgesehen, über weiche der Mantelkörper - und damit die gesamte Schlauchanordnung aufgehängt oder definiert in ihrer räumlichen Bewegbarkeit festgelegt werden können. Diese Fixierstellen können beispielsweise durch Ösen ausgebildet sein. Vorzugsweise sind zwei bis vier derartige Ösen zueinander beabstandet entlang der Schlauchleitung angeordnet.

Gemäß ist der eines Beispiels ist der Mantelkörper derart ausgestaltet, daß dieser einen Behälterabschnitt bildet, in welchen der übrige Teil des Mantelkörpers hineingesteckt werden kann. Dieser in den Mantelkörper integrierte Behälterabschnitt kann als flacher Beutel oder Taschenbereich ausgebildet sein der durch Aufnähen einer flexiblen Lage auf eine Außenfläche des Mantelkörpers gebildet ist. Es ist auch möglich, den überwiegenden Teil des Mantelkörpers in den Innenbereich eines seiner Längsendabschnitte hinein zu zwängen. Hierzu ist vorzugsweise ein Längsabschnitt des Mantelkörpers sackartig ausgebildet.

Ein besonders gewichtssparendes Beispiel des Mantelkörpers wird dadurch erreicht, daß wenigstens eine der isolierenden Lagen als Luftpolster-Lage ausgebildet ist.

Der Mantelkörper ist gemäß einem besonderen Aspekt der vorliegenden Erfindung derart ausgebildet, daß dessen Biegeverhalten in Längsrichtung des Mantelkörpers betrachtet, variiert. So ist es beispielsweise möglich, im maskennahen Bereich des Mantelkörpers eine hohe Flexibilität vorzusehen, wogegen im gerätenahen Bereich der Mantelkörper steifer ausgebildet ist. Das Verformungsverhalten des Mantelkörpers kann hierbei durch Gelenk- und/oder Biegematerialeinlagen bestimmt werden.

Der Mantelkörper ist gemäß eines Beispiels mit einem Knickschutz beispielsweise in Form von Spiral- oder schlangenlinienartig verlaufenden Einlagern versehen. Hierdurch wird es möglich, den in dem Mantelkörper aufgenommenen Atemgasschlauch dünnwandig - ggf. als Einweg-Leicht-Schlauch auszubilden.

Der Mantelkörper kann gemäß eines Beispiels auch länger ausgebildet sein als die eigentliche Schlauchleitung. Hierdurch wird es möglich, durch den Mantelkörper auch Zusatzfunktionselemente abzudecken. So ist es in Verbindung mit dem Mantelkörper möglich, beispielsweise modular an die Schlauchleitung angekoppelte Elemente durch den Mantelkörper abzudecken und zu einer nach außen hin abgeschlossen erscheinenden Einheit zu kombinieren.

Es ist insbesondere möglich, einen rohr- oder schlauchförmig ausgebildeten Luftbefeuchter, ein Gasanalysemodul, einen Gasfluß-Sensor, eine Druckschleuse oder dgl. maskennah modulartig an die Schlauchleitung anzukoppeln und durch den Mantelkörper abzudecken und bedarfsweise zu isolieren. Durch den Mantelkörper sind hierbei in vorteilhafter Weise etwaige Zusatzleitungen elektrischer, optischer, oder auch pneumatischer Art abgedeckt.

Der Mantelkörper bildet hierbei ein flexibles vorzugsweise wärme- und schallisolierendes, die einzelnen Module übergreifendes, und hierbei zusammenfassendes, Gehäuseteil.

Der Mantelkörper kann mit mehreren Öffnungen versehen sein, durch welche Leitungseinrichtungen beispielsweise einer Wasserfalle aus dem Mantelkörper herausgeführt werden können. Diese Öffnungen sind vorzugsweise über eine Deckeleinrichtung verschließbar.

Weitere Einzelheiten der Erfindung ergeben sich aus der nachfolgenden Beschreibung unter Bezugnahme auf die beigefügte Zeichnung. Es zeigen:
- Fig. 1: eine perspektivische Ansicht einer CPAP-Atemgasschlauchanordnung, mit einem, aus einem textilen Weichmaterial gefertigten, Mantelkörper;
- Fig. 2a: eine vereinfachte Schnittansicht, durch einen Abschnitt eines mehrlagig ausgebildeten Mantelkörpers;
- Fig. 2b: eine vereinfachte Schnittansicht, durch einen Abschnitt eines einlagig ausgebildeten Mantelkörpers;
- Fig. 2c: eine vereinfachte Schnittansicht, durch einen Abschnitt eines zweilagig mit einer textilen Decklage ausgebildeten Mantelkörpers;
- Fig. 3: eine perspektivische Darstellung zur Erläuterung einer im Endbereich des flexiblen Mantelkörpers vorgesehenen Fixiereinrichtung;
- Fig. 4: ein Beispiel des flexiblen Mantelkörpers als längsgeteilter Körper einschließlich der sich entlang der entsprechenden Längskante erstreckenden, hier als Klettverschluß ausgebildeten Verbindungseinrichtung.
- Fig.5: eine perspektivische Skizze zur Erläuterung eines Beispiels einer Atemgasschlauchanordnung;
- Fig.6: eine Skizze eines Abschnittes des Mantelkörpers hier mit Ösenabschnitten zur Aufhängung des Mantelkörpers;
- Fig.7: eine Skizze zur Erläuterung eines in den Mantelkörper abschnittsweise einsetzbaren Gelenkeinsatzes;
- Fig.8: eine Skizze einer kartuschenartig ausgebildeten Gebläseeinrichtung (CPAP-Druckquelle) zum Einsatz in den Mantelkörper.

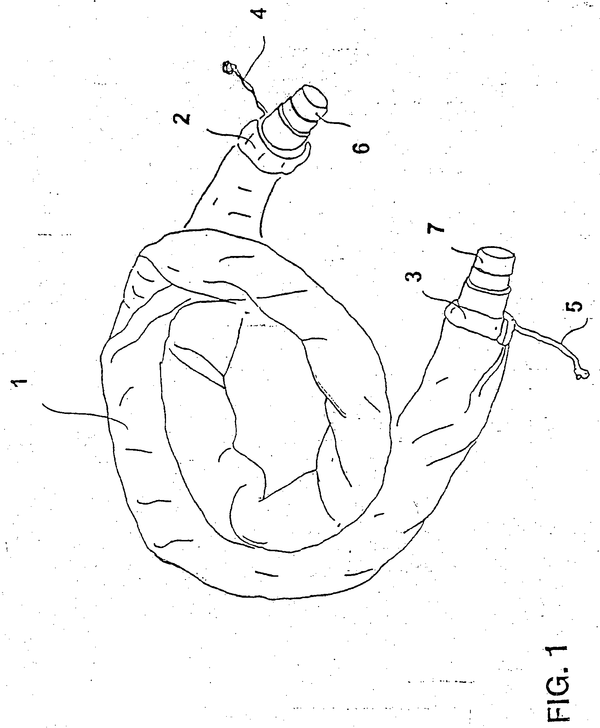

Die in Fig. 1 dargestellte Atemgasschlauchanordnung umfaßt eine hier nicht sichtbare, aus einem spiralverstärkten Kunststoffmaterial gebildete flexible Schlauchleitung und einen aus einem isolierenden Textilmaterial - hier Vliesmaterial - gebildeten isolierenden Mantelkörper 1. Der Mantelkörper 1 ist aus einem schmalen Stoffstreifen gebildet, der entlang einer Längsnaht (Overiocknaht) zu einem Schlauch vernäht ist. Im Endbereich des Mantelkörpers 1 ist bei der hier dargestellten Ausführungsform jeweils ein Hohlsaum 2, 3 ausgebildet, durch welchen jeweils eine Schnur 4, 5 hindurchgeführt ist. Durch entsprechendes Ziehen der Schnüre 4, 5 können die Endbereiche des Mantelkörpers 1 zusammengezogen werden, wodurch auf vorteilhafte Weise der Mantelkörper 1 an der flexiblen Schlauchleitung fixiert ist. Zum Anschluß der flexiblen Schlauchleitung an ein CPAP-Gerät, bzw. an eine Atemmaske sind im jeweiligen Schlauchendbereich Elastomerbuchsen 6, 7 vorgesehen, die elastisch auf eine entsprechend komplementäre Anschlußstruktur auf, bzw. in diese eingesteckt werden können.

Der Mantelkörper 1 ist derart dimensioniert, daß die fiexible Schlauchleitung darin im wesentlichen zwanglos aufgenommen ist. Das hierbei zwischen der Innenfläche des Mantelkörpers 1 und der Außenfläche der flexiblen Schlauchleitung gebildete Luftpolster führt zu einer ohne Gewichtszunahme verbesserten Wärmeisolierung und verleiht der Atemgasschlauchanordnung zudem einen angenehmen Polstereffekt.

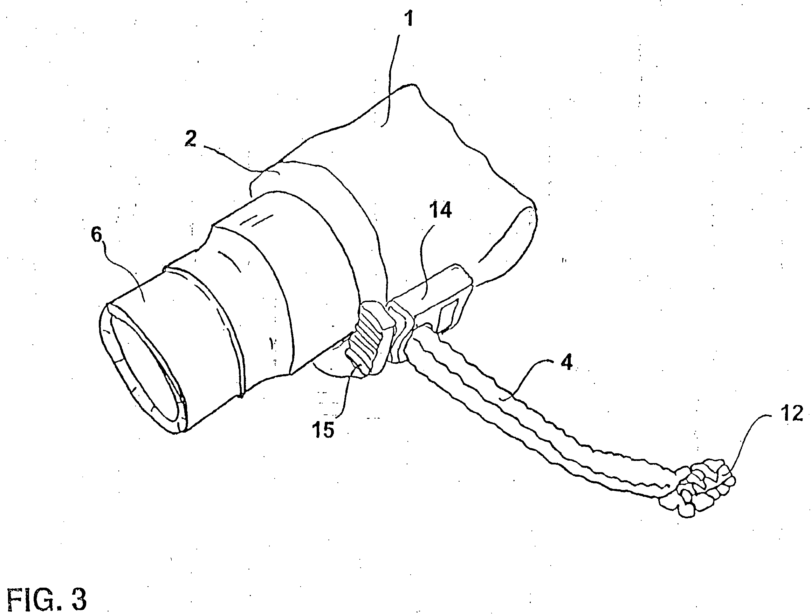

Zur Fixierung der Schnüre 4, 5 in einer entsprechenden Zurrposition sind - wie nachfolgend unter Bezugnahme auf Fig. 3 noch ausführlich erläutert werden wird, vorzugsweise Fixiermittel (hier nicht sichtbar) vorgesehen.

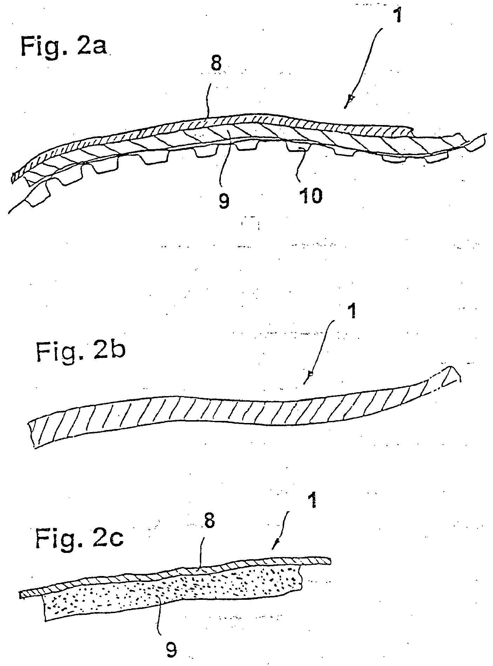

Der Mantelkörper 1 ist vorzugsweise, wie in Fig. 2a andeutungsweise dargestellt, mehrlagig ausgebildet. So kann beispielsweise als oberste, bzw. äußere Decklage eine unter ästhetischen Gesichtspunkten ausgewählte textile Decklage 8 auf einen polsternden und isolierenden Unterbau, der hier aus einer Schaumstofflage 9 und einer Noppenfolie 10 besteht, aufkaschiert werden. Die Verbindung zwischen den einzelnen Lagen kann durch entsprechende Verklebung, durch Nähte oder vorzugsweise durch Wärmekaschieren erfolgen. So ist es beispielsweise möglich, die isolierende Schaumstofflage 9 auf einem Schaumstoffmaterial zu bilden, dessen Deckfläche bei einer vorbestimmten Schmelztemperatur mit der äußeren Lage 8 und der Noppenfolie 10 verschweißt.

Alternativ zu .dem beschriebenen mehrlagigen Aufbau ist es auch möglich, den Mantelkörper 1 aus einem Vollmaterial, vorzugsweise aus einem Vlies- oder Filzmaterial zu bilden, wie dies in Fig. 2b dargestellt ist. Vorzugsweise werden hierbei Materialien verwendet, die eine einfache Reinigung, vorzugsweise einen Kochwaschvorgang gestatten.

In Fig. 2c ist ein Beispiel des Wandungsmaterials des flexiblen Mantelkörpers 1 dargestellt, das hier aus einer polsternden und wärmeisolierenden Schaumstofflage 9 und einer aus einem dekorativen Gewebematerial gebildeten Decklage 8 besteht. Die Verbindung zwischen der Decklage 8 und dem Schaumstoffmaterial 9 ist hier durch einen Flammkaschiervorgang erreicht.

In Fig. 3 ist ein Beispiel einer Fixiereinrichtung zur Fixierung des flexiblen Mantelkörpers 1 im Endbereich des flexiblen Atemgasschlauches dargestellt. Die Fixiereinrichtung umfaßt hier eine durch einen Hohlsaum 2 hindurchgeführte Schnur 4, die im Bereich ihrer herausgeführten Enden mit einem Knoten 12 versehen ist. Durch entsprechendes Ziehen an dem herausgeführten Abschnitt der Schnur 4, bzw. dem Knoten 12 kann das Ende des flexiblen Mantelkörpers 1 zusammengeschnürt werden.

Mittels einer Fixiereinrichtung 14 kann die Schnur 4 in der gespannten Stellung gehalten werden. Durch Betätigung einer hier als Druckknopf 15 ausgebildeten Löseeinrichtung kann die Schnur 4 gelockert und hierbei das zunächst eingeschnürte Ende des Mantelkörpers 1 geweitet werden. Eine besonders sichere Fixierung des Mantelkörpers 1 an der flexiblen Schlauchleitung kann dadurch erreicht werden, daß im Bereich des hier als elastomere Buchse 6 ausgebildeten Abschlußelementes eine Struktur ausgebildet ist, die mit dem entsprechenden Endabschnitt des Mantelkörpers 1 in Eingriff bringbar ist. Beispielsweise kann die elastomere Buchse 6 mit einer Umfangsnut oder einem Umfangswulst versehen sein, an welchem der entsprechend zusammengeschnürte textile Mantelkörper 1 fixierbar ist.

Alternativ zu der hier dargestellten Zurranordnung ist es auch möglich, das Ende des Mantelkörpers 1, beispielsweise über eine Klettverschlußeinrichtung oder Schnallen an der flexiblen Schlauchleitung (nicht dargestellt) zu fixieren.

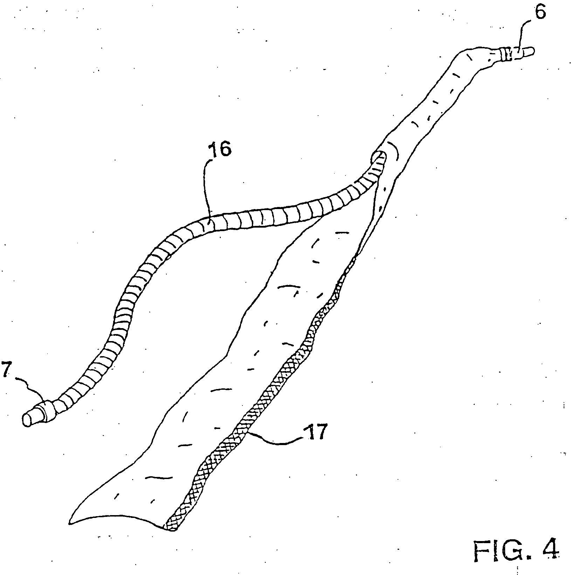

In Fig. 4 ist ein Beispiel der Atemgasschlauchanordnung dargestellt, die hier eine flexible Schlauchleitung 16 aufweist, an deren Endbereich die elastomeren Buchsen 6, 7 vorgesehen sind. Abweichend von der vorangehend in Verbindung mit der in Fig. 1 beschriebenen Ausführungsform ist hier der flexible Mantelkörper 1 aus einem flexiblen Textilstreifen gebildet, der um die flexible Schlauchleitung 16 herumgeführt und entlang seiner Längskante mit einer Verbindungseinrichtung 17 versehen ist, so daß der um die Schlauchleitung 16 herumgeführte Materialstreifen ebenfalls schlauchartig die flexible Schlauchleitung 16 umgibt. In besonders vorteilhafter Weise ist hierzu im Bereich einer der Längskanten des Textilmaterialstreifens ein Klettverschlußband vorgesehen.

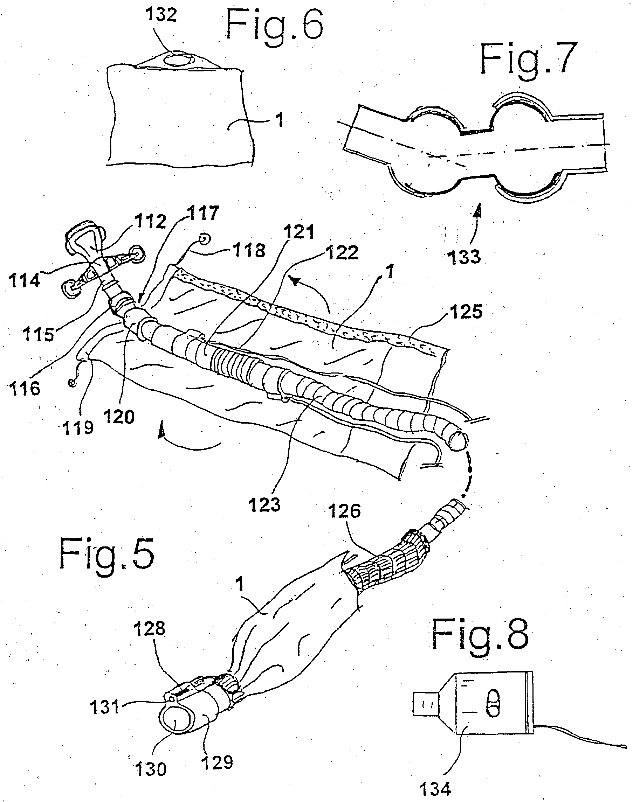

In Fig.5 ist ein Beispiel der in Verbindung mit dem Mantelkörper 1 gebildeten Atemgasleitungseinrichtung einschließlich Atemmaske 112 dargestellt.

Die Atemmaske 112 ist über ein, in einen Stirnabstandshalter 114 integriertes Auswaschventil 115 an ein Gelenkstück 116 angeschlossen. Im Bereich des Gelenkstückes 116 ist eine Umfangsnut 117 ausgebildet, in welcher mittels eines Zurrbandes 118 ein vorderer Bundbereich 119 des Mantelkörpers 1 festlegbar ist.

An das Gelenkstück 116 schließt sich ein Atemgasstrom-Meßglied 120 an, das über eine hier nicht sichtbare Meßleitung mit einer Atemgasdruckquelle, beispielsweise einem CPAP-Gerät verbindbar ist.

Die Atemgasleitungseinrichtung umfaßt weiterhin einen Atemgasbefeuchter 121 der über eine Befeuchtungsleitung 122 mit einer Wasserquelle verbindbar ist. Der Atemgasbefeuchter 121 ist als flexibles Rohrstück ausgebildet und zwischen Atemmaske 112 und dem überwiegenden Teil des Atemgasschlauches 23 angeordnet.

Im Bereich des Atemgasbefeuchters 121 ist der Mantelkörper 1 mit einer Superisolations-Folie versehen, so daß im Befeuchterbereich eine hohe Wärmeisloationswirkung erreicht wird. Hierdurch wird es möglich, die zur Verdunstung des Befeuchtungswassers erforderliche Wärme ausschließlich - oder zumindest überwiegend - aus der Atemluft zu beziehen.

Der Mantelkörper 1 fügt sich in Schließstellung schlauchartig über die aus mehreren Modulen zusammengesetzte Atemgasleitung und schließt diese nach außen ab. An dem Mantelkörper 1 ist bei dem gezeigten Ausführungsbeispiel ein Klettverschlußband 125, vorgesehen.

Die Atemgasleitung umfaßt weiterhin einen Schläuchschalldämpfer 126 der hier eine Länge von ca. 80 bis 120 cm aufweist und eine weitere Absorption etwaiger Geräusche einer Atemgasdruckquelle ermöglicht. Auch dieser Schalldämpfer 126 wird bei der hier gezeigten Ausführungsform noch von dem Mantelkörper 1 ummantelt.

In den Mantelkörper 1 ist eine Heizeinrichtung integriert. Die Spannungsversorgung dieser Heizeinrichtung erfolgt über Kontaktelemente 128, die in einen Anschlußstecker 129 integriert sind. Der Anschlußstecker 129 umfaßt einen Atemgasdurchgangsquerschnitt 130 sowie einen Druckmeßschlauchanschlußquerschnitt 131.

In Fig.6 ist ein Abschnitt des Mantelkörpers 1 dargestellt, der hier mit einer Öse 132 versehen ist, über weiche der Mantelkörper 1 aufgehängt werden kann.

In Fig.7 ist ein Gelenkgliederelement 133 dargestellt, das koaxial zur Atemgasleitung in den Mantelkörper 1 einsetzbar ist. Das Gelenkgliederelement 133 umfaßt eine Vielzahl an Gelenkgliedern, die derart eng sitzend miteinander gekoppelt sind, daß die Atemgasleitungsanordnung in eine gewünschte Form biegbar ist.

In Fig.8 ist eine Gebläseeinrichtung 134 dargestellt, die hinsichtlich ihrer äußeren Abmessungen derart bemessen ist, daß diese ebenfalls noch in den Mantelkörper.1 einsetzbar ist. Hierdurch wird es möglich, eine schal- oder schlangenartig ausgebildete, modulartig auf den jeweiligen Anwender abgestimmte CPAP-Einrichtung zu schaffen.

## Patentansprüche

1. Anschlusselement zum Verbinden einer Gebläseeinrichtung mit einer Atemgasschlauchanordnung mit einer Heizeinrichtung zur Zufuhr eines Atemgases zu einer Person, wobei das Anschlusselement ausgebildet ist, um die Schlauchleitung mit einer Gebläseeinrichtung zu verbinden, und wobei das Anschlusselement als Anschlussstecker ausgebildet ist, der einen Atemgasdurchgangsquerschnitt sowie Kontaktelemente bereitstellt, über die die Heizeinrichtung mit Spannung versorgt wird.

2. Anschlusselement nach Anspruch 1, wobei die Kontaktelemente in den Anschlussstecker integriert sind.

3. Anschlusselement nach Anspruch 1 oder 2, wobei der Anschlussstecker femer Druckmessschlauchanschlussquerschnitt aufweist.

4. Flexible Atemgasschlauchanordnung zur Zufuhr eines Atemgases zu einer Person, aufweisend:
eine flexible Schlauchleitung;
eine Heizeinrichtung zum Erwärmen des durch die Schlauchleitung (123) geförderten Gases;
an den Enden der Schlauchleitung (123) vorgesehene Anschlusselemente, um die Schlauchleitung (123) mit einer Atemmaske (112) bzw. einer Gebläseeinrichtung zu verbinden; wobei
das Anschlusselement zur Verbindung mit einer Gebläseeinrichtung als Anschlussstecker gemäß einem der Ansprüche 1 bis 3 ausgebildet ist.

5. Atemgasschlauchanordnung nach Anspruch 4, **gekennzeichnet durch** einen flexiblen Mantelkörper (1), der die flexible Schlauchleitung (16) umgibt und sich entlang der Schlauchleitung (16) erstreckt.

6. Atemgasschlauchanordnung nach Anspruch 5, **dadurch gekennzeichnet, dass** der flexible Mantelkörper (1) aus einem wärmeisolierenden Material gebildet ist.

7. Atemgasschlauchanordnung nach Anspruch 5 oder 6, **dadurch gekennzeichnet, dass** der flexible Mantelkörper (1) aus einem Fleecematerial gebildet ist.

8. Atemgasschlauchanordnung nach wenigstens einem der Ansprüche 5 bis 7, **dadurch gekennzeichnet, dass** der flexible Mantelkörper (1) mehrere Materialschichten (8, 9, 10) aufweist.

9. Atemgasschlauchanordnung nach wenigstens einem der Ansprüche 5 bis 8, **dadurch gekennzeichnet, dass** der flexible Mantelkörper (1) im Bereich seiner Stirnenden mit einer Verschlusseinrichtung (2, 4; 3, 5) versehen ist.

10. Atemgasschlauchanordnung nach Anspruch 9, **dadurch gekennzeichnet, dass** die Verschlusseinrichtung durch eine Zurr- oder Klettverschlusseinrichtung gebildet ist.

11. Atemgasschlauchanordnung nach wenigstens einem der Ansprüche 5 bis 10, **dadurch gekennzeichnet, dass** der flexible Mantelkörper (1) in Längsrichtung geteilt ist.

12. Atemgasschlauchanordnung nach wenigstens einem der Ansprüche 5 bis 11, **dadurch gekennzeichnet, dass** der flexible Mantelkörper (1) eine sich entlang einer Seitenkante erstreckende Klettverschlussverbindungseinrichtung (17) aufweist.

13. Vorrichtung zur Zufuhr eines Atemgases zu einem Patienten mit einer Fördereinrichtung zur Förderung des Atemgases, einer Atemmaske und einer flexiblen Atemgasschlauchleitung, nach einem der Ansprüche 4 bis 12 zur Koppelung der Atemmaske mit der Fördereinrichtung.

14. CPAP-Vorrichtung, aufweisend:
eine Fördereinrichtung zur Förderung von Atemgas über eine flexible Atemgasschlauchanordnung, insbesondere nach einem der Ansprüche 4 bis 12, zu einem Patienten;
eine elektronische Regeleinrichtung zum Abstimmen des Förderdruckes der Fördereinrichtung; und
ein Anschlussstruktur, zum Verbinden mit einem Anschlusselement gemäß Anspruch 1,2 oder 3, wobei die Anschlussstruktur eine Spannungsversorgung aufweist, um elektrische Spannung an die Kontaktelemente des Anschlusssteckers bereitzustellen, um Spannung an die Heizeinrichtung bereitzustellen, um das durch die Atemgasschlauchanordnung (123) geförderte Gas zu erwärmen.

## Claims

1. A connector element for connecting a blower means with a respiratory gas tube arrangement comprising heating means for supplying a respiratory gas to a person, wherein the connector element is adapted for connecting the tube with a blower means, and wherein the connector element is configured as a connector plug providing a respiratory gas passage cross-section as well as contact elements for supplying the heating means with power.

2. Connector element according to claim 1, wherein the contact elements are integrated into the connector plug.

3. Connector element according to claim 1 or 2, wherein the connector further comprises a pressure measuring tube connection cross-section.

4. A flexible respiratory gas tube arrangement for supplying a respiratory gas to a person, comprising:
a flexible tube;
heating means for heating the gas supplied through the tube (123);
connector elements provided at the ends of the tube (123) for connecting the tube (123) with a breathing mask (112) or a blower means; wherein
the connector element for connection with a blower means is configured as a connector plug according to any one of claims 1 to 3.

5. A respiratory gas tube arrangement according to claim 4, **characterized by** a flexible sheathing body (1) which surrounds the flexible tube (16) and extends along the tube (16).

6. The respiratory gas tube arrangement according to claim 5, **characterized in that** the flexible sheathing body (1) is formed of a heat-insulating material.

7. The respiratory gas tube arrangement according to claim 5 or 6, **characterized in that** the flexible sheathing body (1) is formed of a fleece material.

8. The respiratory gas tube arrangement according to at least one of claims 5 to 7, **characterized in that** the flexible sheathing body (1) comprises a plurality of material layers (8, 9, 10).

9. The respiratory gas tube arrangement according to at least one of claims 5 to 8, **characterized in that** the flexible sheathing body (1) comprises closure means (2, 4; 3, 5) in the region of its face ends.

10. The respiratory gas tube arrangement according to claim 9, **characterized in that** the closure means is formed by a lashing or hook and loop fastener means.

11. The respiratory gas tube arrangement according to at least one of claims 5 to 10, **characterized in that** the flexible sheathing body (1) is divided in the longitudinal direction.

12. The respiratory gas tube arrangement according to at least one of claims 5 to 11, **characterized in that** the flexible sheathing body (1) comprises a hook and loop fastener joining means (17) extending along a side edge.

13. A device for supplying a respiratory gas to a patient, comprising a supply means for supplying the respiratory gas, a breathing mask and a flexible respiratory gas tube, according to any one of claims 4 to 12, for coupling the breathing mask with the supply means.

14. A CPAP device comprising:
a supply means for supplying respiratory gas through a flexible respiratory gas tube arrangement, in particular according to any one of claims 4 to 12, to a patient;
an electronic control means for adjusting the supply pressure of the supply means; and
a connector structure for connection with a connector element according to claim 1, 2, or 3, wherein the connector structure comprises a power supply for supplying the contact elements of the connector plug with power in order to supply power to the heating means so as to heat the gas supplied through the respiratory gas tube arrangement (123).

## Revendications

1. Élément de connexion pour le raccordement d'un système de ventilation à un dispositif de tuyau pour gaz respiratoire équipé d'un système de chauffage et destiné au refoulement d'un gaz respiratoire vers une personne, où ledit élément de connexion est prévu pour raccorder le dispositif de tuyau pour gaz respiratoire à un système de ventilation, et où ledit élément de connexion est réalisé comme fiche de connexion fournissant une section de passage de gaz respiratoire ainsi que des éléments de contact au moyen desquels le système de chauffage est alimenté en tension.

2. Élément de connexion selon la revendication 1, où les éléments de contact sont intégrés à la fiche de connexion.

3. Élément de connexion selon la revendication 1 ou la revendication 2, où la fiche de connexion présente en outre une section de connexion à un tuyau de mesure de pression.

4. Dispositif de tuyau flexible pour gaz respiratoire destiné au refoulement d'un gaz respiratoire vers une personne, comportant :
un tuyau flexible ;
un système de chauffage pour le chauffage du gaz refoulé par la tuyau flexible (123) ;
des éléments de connexion prévus aux extrémités de la tuyau flexible (123) pour raccorder ladite tuyau flexible (123) à un masque respiratoire (112) ou à un système de ventilation ; où
l'élément de connexion pour le raccordement à un système de ventilation étant réalisé comme fiche de connexion selon l'une des revendications 1 à 3.

5. Dispositif de tuyau pour gaz respiratoire selon la revendication 4, **caractérisé par** une enveloppe flexible (1) entourant le tuyau flexible (16) et s'étendant le long du tuyau flexible (16).

6. Dispositif de tuyau pour gaz respiratoire selon la revendication 5, **caractérisé en ce que** l'enveloppe flexible (1) est en matériau thermo-isolant.

7. Dispositif de tuyau pour gaz respiratoire selon la revendication 5 ou la revendication 6, **caractérisé en ce que** l'enveloppe flexible (1) est en laine polaire.

8. Dispositif de tuyau pour gaz respiratoire selon au moins une des revendications 5 à 7, **caractérisé en ce que** l'enveloppe flexible (1) comprend plusieurs couches de matériau (8, 9, 10).

9. Dispositif de tuyau pour gaz respiratoire selon au moins une des revendications 5 à 8, **caractérisé en ce que** l'enveloppe flexible (1) est pourvue d'un système de fermeture (2, 4 ; 3, 5) au niveau de ses extrémités frontales.

10. Dispositif de tuyau pour gaz respiratoire selon la revendication 9, **caractérisé en ce que** le système de fermeture est formé par un dispositif de fermeture à cordon ou à ruban agrippant.

11. Dispositif de tuyau pour gaz respiratoire selon au moins une des revendications 5 à 10, **caractérisé en ce que** l'enveloppe flexible (1) est divisée dans le sens de la longueur.

12. Dispositif de tuyau pour gaz respiratoire selon au moins une des revendications 5 à 11, **caractérisé en ce que** l'enveloppe flexible (1) comporte un système de raccordement par fermeture à ruban agrippant (17) s'étendant le long d'un bord latéral.

13. Installation pour le refoulement d'un gaz respiratoire vers un patient, avec un dispositif de refoulement pour le refoulement du gaz respiratoire, un masque respiratoire et un tuyau flexible selon l'une des revendications 4 à 12 pour l'accouplement du masque respiratoire au dispositif de refoulement.

14. Installation de ventilation en pression continue, comprenant :
un dispositif de refoulement pour le refoulement de gaz respiratoire vers un patient par un dispositif de tuyau flexible pour gaz respiratoire, en particulier selon l'une des revendications 4 à 12 ;
un dispositif de régulation électronique pour le réglage de la pression de refoulement du dispositif de refoulement ; et
une structure de connexion pour le raccordement à un élément de connexion selon la revendication 1, 2 ou 3, ladite structure de connexion présentant une tension d'alimentation pour fournir une tension électrique aux éléments de contact de la fiche de connexion, pour fournir une tension au système de chauffage afin de chauffer le gaz refoulé par le dispositif de tuyau pour gaz respiratoire (123).
